# EUROPEAN PATENT SPECIFICATION

(11) **EP 1 363 949 B1**
(45) Date of publication and mention of the grant of the patent: **26.09.2007**
(21) Application number: 01996559.9
(22) Date of filing: 29.10.2001
(51) Int. Cl.: C07K 14/72, G01N 33/68

(54) **FUNCTIONAL FRAGMENT OF THE LEPTINE RECEPTOR**
FUNKTIONELLES FRAGMENT DES LEPTINREZEPTORS
FRAGMENT FONCTIONNEL DU RECEPTEUR DE LA LEPTINE

(30) Priority: 14.11.2000 EP 00204001; 15.11.2000 US 248970 P
(43) Date of publication of application: 26.11.2003
(73) Proprietor: Vlaams Interuniversitair Instituut voor Biotechnologie vzw., 9052 Zwijnaarde (BE)
(72) Inventor: EYCKERMAN, Sven, B-9000 Gent (BE); TAVERNIER, Jan, B-9860 Balegem (BE); ZABEAU, Lennart, B-9000 Gent (BE)
(86) International application number: PCT/EP2001/012569
(87) International publication number: WO 2002/040543

(56) References cited:
- WO-A-97/26335
- WO-A-99/40946
- LEE G -H ET AL: "ABNORMAL SPLICING OF THE LEPTIN RECEPTOR IN DIABETIC MICE" NATURE,GB,MACMILLAN JOURNALS LTD. LONDON, vol. 379, 15 February 1996 (1996-02-15), pages 632-635, XP002030818 ISSN: 0028-0836
- S. GISSELBRECHT: "The CIS/SOCS proteins" EUROPEAN CYTOKINE NETWORK, [Online] December 1999 (1999-12), pages 463-470, XP002171849 Retrieved from the Internet: <URL:http://www.john-libbey-eurotext.fr/ar ticles/ecn/10/4/463-70/> [retrieved on 2001-07-12]
- BONNEFOY-BERARD N ET AL: "VAV: FUNCTION AND REGULATION IN HEMATOPOIETIC CELL SIGNALING" STEM CELLS,US,ALPHAMED PRESS, DAYTON, OH, vol. 14, 1996, pages 250-268, XP000864379 ISSN: 1066-5099

## Description

The present invention relates to a functional fragment of a receptor, important in signaling. Particularly, the present invention relates to a functional fragment of the leptin receptor, involved in SOCS3, CIS and/or Vav signaling.

Leptin, an adipocyte derived hormone, delivers its appetite suppressing signals by passing the blood-brain barrier and by binding to the specific signaling form of its receptor in certain nuclei of the hypothalamus. In this way it constitutes a feedback mechanism regulating adipose tissue mass. Mutations within the leptin system result in a marked obese phenotype and impaired endocrinological functioning (Zhang *et al.,* 1994, Chen *et al.,* 1996). Although the long signaling form of the leptin receptor shows high expression levels in these hypothalamic nuclei, it is also expressed in several peripheral tissues including lung, liver, lymph nodes and gonads (Tartaglia *et al.,* 1995, Ghilardi *et al.,* 1996). This leads to the involvement of leptin in several peripheral functions, making it a typical pleiotropic cytokine.

Until today the therapeutic use of leptin as a weight-reducing agent remains limited (Heymsfield *et al.,* 1999). It is observed that in most obese people a strong correlation exists between adipose mass and leptin levels, a phenomenon often explained by leptin resistance (Maffei *et al.,* 1995). A number of possible explanations for this resistance have been suggested: a saturable transport through the blood-brain barrier resulting in a limited leptin activity in the hypothalamus (Schwartz *et al.,* 1996, El Haschimi *et al.,* 2000), cross-talk with the glucocorticoid system (Zakrzewska *et al.,* 1997), or defects at the leptin receptor level, such as elevated expression of the signaling inhibitor SOCS3 (Bjorbaek *et al.,* 1998).

Recently, it became clear that leptin is not only playing a role in regulating food intake, but functions in angiogenesis (Sierra-Honigmann *et al.,* 1998) and in invasiveness of kidney and colonic epithelial cells (Attoub *et al.,* 2000) have been demonstrated.

Being a member of the type 1 cytokine receptor family, the leptin receptor is activated by cross-phosphorylation of associated JAK kinases, most likely JAK2 and/or JAK1 (Bjorbaek *et al.,* 1997, Banks *et al.,* 2000). Activation of the leptin receptor leads to recruitment of signaling molecules containing phosphotyrosine binding SH2 modules. Signal Transducers and Activators of Transcription (STAT) molecules (Baumann *et al.,* 1996, Vaisse *et al.,* 1996) and the receptor-associated SH2-containing phosphatase SHP-2 (Carpenter *et al.,* 1998, Li and Friedman, 199) are both recruited in the activated leptin receptor complex. Leptin mediated activation of Mitogen Activated Protein Kinases (MAPK) and Insulin Receptor Substrate 1 (IRS-1) has been shown in various cell systems (Cohen *et al.,* 1996, Bjorbaek *et al.,* 1997, Takahashi *et al.,* 1997, Banks *et al.,* 2000). Promotion of invasiveness by leptin seems to be mediated by phosphoinositide 3-kinase, Rho- and Rac-dependent signaling pathways (Attoub *et al.,* 2000)

Leptin rapidly induces Cytokine-Inducible SH2-containing protein (CIS), both in vitro and in vivo. Leptin can also strongly and rapidly induce the production of the signal transduction inhibitor Suppressor of Cytokine Signaling 3 (SOCS3) in various cell types and *in vivo* (Bjorbaek *et al.,* 1998, Emilsson *et al.,* 1999, Waelput *et al.,* 2000). Both CIS and SOCS3 are members of an expanding family of SH2 containing proteins which are typically built up of a pre-SH2 domain, a central SH2 domain, and a highly conserved SOCS box sequence. The latter motif is also found in a number of other signaling molecules (Hilton *et al.,* 1998) and seems to be connected with proteasome function (Zhang *et al.,* 1999). The observation that the leptin resistant *A^{y}la* mutant mice strain shows elevated SOCS3 levels makes this protein a possible mediator of leptin resistance (Bjorbaek *et al.,* 1998).

Recently it has been shown that a tyrosine recruitment site within gp130, the signaling component of the IL-6 complex, is required for binding and thus for the inhibitory activity of SOCS3 (Nicholson *et al.,* 2000, Schmidt *et al.,* 2000). This is in contrast to SOCS1, which binds directly to JAK kinases and directly inhibits their kinase activity (Yasukawa *et al.,* 1999). Similar results have been obtained for the insulin receptor and the erythropoietin receptor (Emanuelli *et al.,* 2000, Sasaki *et al.,* 2000).

In previous studies, it has been shown that leptin induces two gene sets in the PC12 rat pheochromocytoma cell line stably expressing the mouse leptin receptor. Many of these genes appear to be regulated *in vivo* (Waelput *et al.,* 2000). Using a mutational approach it was shown that in the mouse leptin receptor residue Y985 is involved in a negative feedback signal, and furthermore that this effect is more pronounced when mutated in concert when another tyrosine, Y1077 (Eyckerman *et al.,* 1999). However, phosphorylation of this tyrosine has never been demonstrated, suggesting that this site was playing only a minor role. Surprisingly, we found that a short functional fragment around said Y1077 is sufficient for SOCS3 and CIS binding and/or signaling. Moreover, we were able to demonstrate that said functional fragment is also sufficient for Vav signaling. Vav seems to be a general signaling molecule, but has never been shown to be involved in leptin signaling. Said short functional sequence is extremely conserved, which makes it an attractive target for pharmaceutical compositions modulating SOCS3, CIS and/or Vav mediated signaling in general, and leptin induced signaling in particular.

A first aspect of the invention is to provide a functional fragment of a receptor, comprising a sequence selected from the group consisting of SEQ ID N° 1, SEQ ID N° 2, SEQ ID N° 3 and SEQ ID N° 4, preferably essentially consisting of a sequence selected from the group consisting of SEQ ID N° 1, SEQ ID N° 2, SEQ ID N° 3 and

SEQ ID N° 4, more preferably consisting of a sequence selected from the group consisting of SEQ ID N° 1, SEQ ID N° 2, SEQ ID N° 3 and SEQ ID N° 4. Preferably, said functional receptor fragment essentially consists of a sequence consisting of SEQ ID N° 5 or SEQ ID N° 6, more preferably, said functional receptor fragment consists of a sequence consisting of SEQ ID N° 5 or SEQ ID N° 6. Preferentially, said functional fragment is involved in SOCS3, CIS and/or Vav signaling when it consists of SEQ ID N°1, SEQ ID N° 2 or SEQ ID N° 6, and involved in Vav signaling when it consists of SEQ ID N° 3, SEQ ID N° 4 or SEQ ID N° 5. In the cases where the sequence comprises a tyrosine residue, the functional fragment may be phosphorylated on said residue or not. In case of SOCS3 and CIS signaling, said tyrosine residue is preferentially phosphorylated. The phosphorylation may be carried out by an associated kinase, e.g. a JAK kinase such as JAK2, that binds on another domain of the receptor, or by a kinase activity inherent to the receptor itself. Indeed, although the functional fragment is derived from the cytoplasmic domain of the leptin receptor, it is evident for the person skilled in the art that it can be incorporated in other receptors such as, as a non limiting example, the cytokine receptor family or the protein tyrosine kinase family, where it can function as recruitment site for signaling molecules.

It is a further aspect of the invention to use said functional fragment to modulate ligand induced signaling. Indeed, said functional fragment is involved in SOCS3 signaling, which is know to be a suppressor of cytokine signaling. Incorporation of said functional fragment in a cytokine receptor is therefore expected to modulate the cytokine induced signaling. A similar effect is expected in other receptors due to the involvement of Vav signaling. A preferred embodiment is the use of said functional fragment to modulate leptin induced signaling. Such modulation may not only be useful in food intake disorders and regulation of body weigth, but also in other leptin-mediated phenomena such as angiogenesis, wound healing, and susceptibility to digestive cancers.

Especially the modulation of the latter may be important, as Vav has been shown to bind to said functional fragment, and Vav is know to act as a guanosine nucleotide exchange factor for Rac-1, which is involved in leptin-induced invasiveness of kidney and colonic epithelial cells (Attoub *et al.,* 2000)

Another aspect of the invention is the use of said functional fragment to screen compounds that interfere with the binding of said functional fragment with a signaling molecule. Indeed, a receptor can be constructed, wherein said functional fragment is the only functional fragment, such as, as a non-limiting example, a mouse leptin receptor in which the tyrosine residues at positions 985 and 1138 have been replaced by a phenylalanine. This receptor, comprising the functional fragment, may be expressed in a suitable host cell. Binding of a ligand on said receptor may induce a reporter gene, whereby the induction is mediated by binding of a signaling molecule on the functional fragment. By bringing said host cell in contact with a library of small molecules, or by transfecting said host cell by a library encoding potentially inhibiting peptides, compounds inhibiting the binding of a signaling molecule with said functional fragment can be identified by selecting those host cells that do not show an induction of the reporter gene anymore upon contacting said receptor with its ligand. Alternatively, the induction of the signaling pathway as such may be monitored, instead of the induction of a reporter gene.

Still another aspect of the invention is a compound, isolated by the use of said functional fragment. Said compound can be, as a non-limiting example, a soluble peptide having the same sequence as said functional fragment, or a peptido-mimetic thereof, an antibody, binding to said functional fragment, or an antibody binding to a domain in SOCS3, CIS or Vav that binds directly or indirectly to said functional fragment.

### Definitions

*Functional fragment* as used here is a peptide or polypeptide, optionally carrying one or more modifications, which, when integrated in a suitable receptor molecule, will function as a binding site for one or more signaling molecules. This binding may be either constitutive or ligand induced.

*Modification* as used here may be any modification of an amino acid, known to the person skilled in the art, such as, as a non-limiting example, phosphorylation, glycosylation or ubiquitinylation.

*Essentially consisting* as used here means that the cited SEQ ID is embedded in a total functional fragment of only 50 amino acids, preferably only 30 amino acids, even more preferably only 20 amino acids.

*Compound* means any chemical or biological compound, including simple or complex organic or inorganic molecules, peptides, peptido-mimetics, proteins, antibodies, carbohydrates, nucleic acids or derivatives thereof.

*Bind(ing)* means any interaction, be it direct or indirect. A direct interaction implies a contact between the binding partners. An indirect interaction means any interaction whereby the interaction partners interact in a complex of more than two compounds. The interaction can be completely indirect, with the help of one or more bridging molecules, or partly indirect, where there is still a direct contact between the partners, which is stabilized by the additional interaction of one or more compounds

*Signaling molecule* as used here means any molecule that is involved in the transfer or the inhibition of the transfer of an activated receptor to a reporter gene. In that respect, a molecule that is not directly involved in signaling itself, but that, by binding on the receptor can inhibit another molecule from binding and inducing the signaling pathway is also considered as a signaling molecule.

*Reporter gene* is any gene that leads to a detectable signal and can be, as a non-limiting example, an antibiotic resistance gene, a toxin gene resulting in cell death, a gene encoding a fluorescent protein such as GFP, or a gene encoding an enzyme activity such as β-galactosidase. The coding sequence is placed under control of a suitable promoter, i.e. a promoter that is induced by binding of a ligand to the receptor and consequent induction of the reporter pathway.

### Brief description of the figures

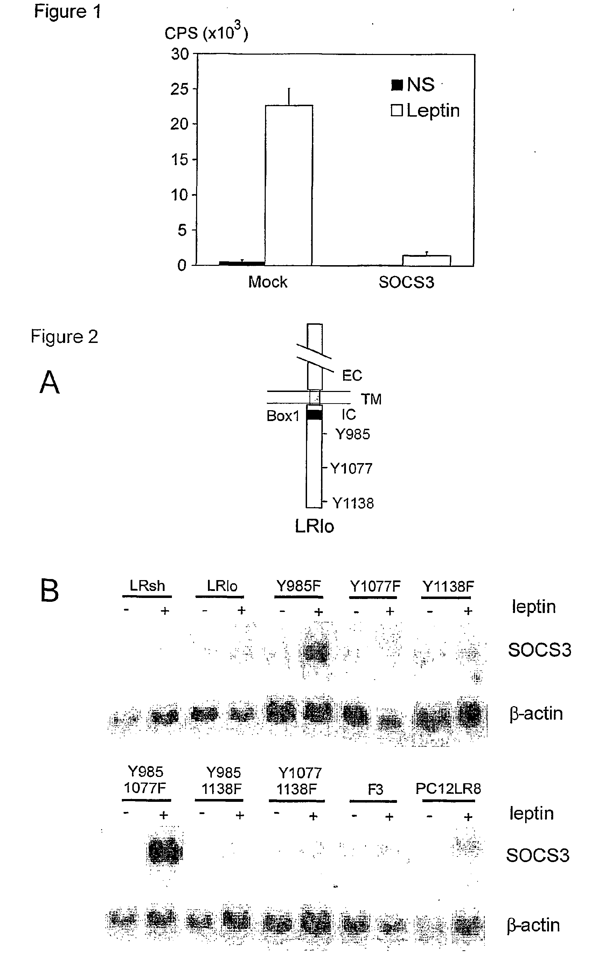

### Figure 1: Expression of SOCS3 leads to inhibition of leptin-induced rPAP1 expression in PC12 cells

PC12 LR8 cells were co-transfected with a plasmid encoding SOCS3 (pMET7fSOCS3) or empty vector (mock, pMET7), and a pGL3-rPAP1luci reporter construct. 48h after transfection, the cells were stimulated with leptin or left untreated. Luciferase activity was measured in triplicate after 24h. CPS: absolute luminescence counts per second; NS: non-stimulated negative control.

### Figure 2: (A) Schematic representation of the long isoform of the murine leptin receptor

(LRIo), showing extracellular (EC), transmembrane (TM) and intracellular (IC) parts. The Box1 motif and the three tyrosines involved in signal transduction are also shown. **(B) Y985F and Y985/1077F, but not Y1077F mutations in the leptin receptor lead to augmented levels of SOCS3 mRNA**. PC12 cells were transiently transfected with plasmids encoding different leptin receptor variants and were stimulated with leptin for 24h. Northern blot analysis was performed on lysates using a rat SOCS3 probe. A β-actin probe was used for mRNA quantitation. Induction of SOCS3 is also shown for PC12LR8 cells stably expressing the murine leptin receptor. LR: leptin receptor; sh: short isoform ; lo: long isoform ; F3: all cytoplasmic tyrosines mutated to phenylalanine.

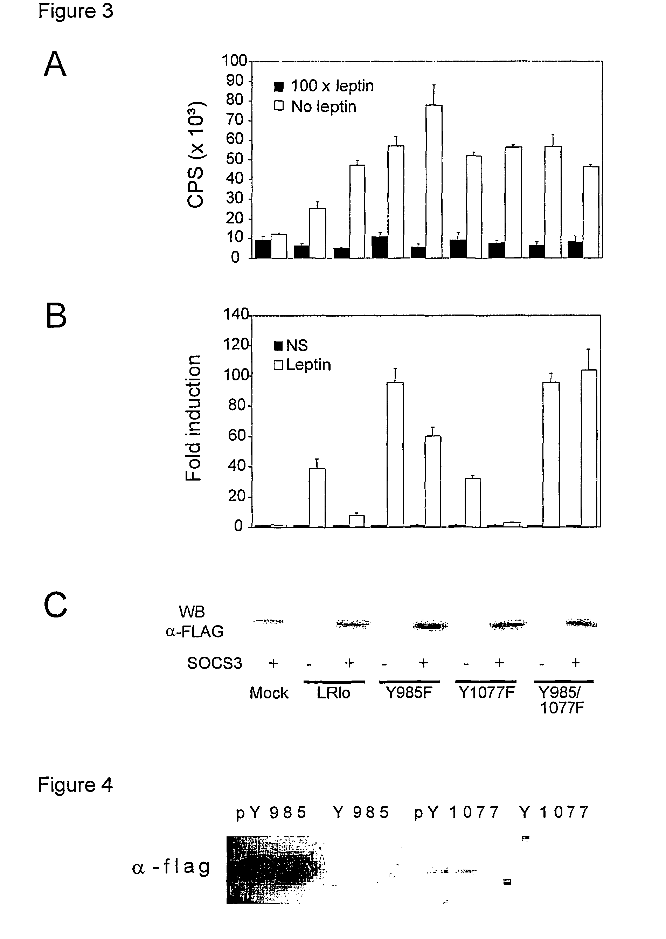

### Figure 3: SOCS3-mediated inhibition of leptin signaling is critically dependent on the presence of either Y985 or Y1077 in the murine leptin receptor

Hek293T cells were transiently co-transfected with plasmids encoding different leptin receptor variants, SOCS3 or empty vector, together with the pXP2d2-rPAP1luci reporter construct. (**A**) LR expression levels were measured on transfected cells by incubation for 90' with Leptin-SEAP fusion protein with or without excess leptin (100x). Bars show mean value and SD values of triplicate measurements. (**B**) The transfected cells were either stimulated for 24h with leptin or left untreated. Luciferase measurements were performed in triplicate and were normalized by co-transfection with the pUT651 β-galactosidase construct and a β-galactosidase activity assay. Results are shown in fold induction. (C) Western blot analysis of SOCS3 expression. The FLAG-tagged SOCS3 protein was revealed in lysates of transfected cells using anti-FLAG antibody. CPS: absolute luminescence counts per second; LRlo: leptin receptor long isoform.

### Figure 4: SOCS3 binding to peptides matching leptin receptor Y985 and Y1077 sites is phosphorylation dependent

Lysates of Hek293T cells overexpressing FLAG-tagged SOCS3 protein were incubated with peptides matching leptin receptor Y985 and Y1077 sites, either phosphorylated or not. Specific binding of SOCS3 was revealed by immunoblotting using an anti-FLAG antibody.

### Figure 5: CIS interacts with the Y985 and Y1077 motifs in the leptin receptor

HEK293T cells were transiently cotransfected with the pXP2d2-rPAP1luci, pMG1-CIS and pMET7-LR mutants as indicated. 36 hours post transfection, cells were stimulated with leptin for 24 hours (open bars) or were left untreated (solid bars). Luciferase activity is indicated as the mean of triplicate measurements +/- SD. β-galactosidase activity from pUT651 was used to normalise for variations in transfection efficiencies. CPS: light counts per second.

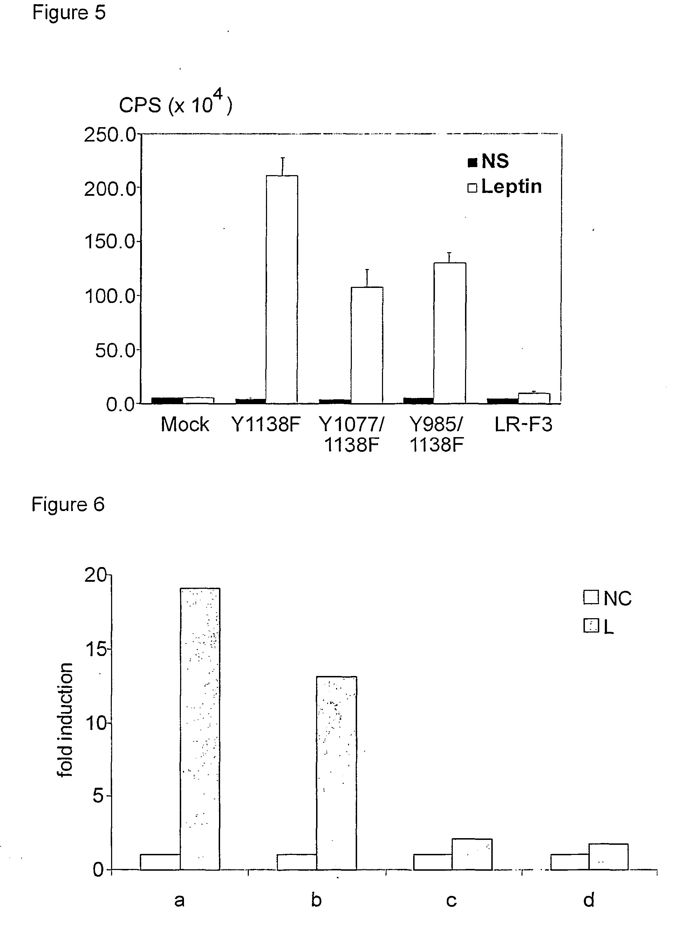

### Figure 6: Functionality of mLR F3 deletion and point mutants

Cells were transfected with
a. pMET7-mLR F3 + pMG1-VavS + pXP2d2-rPAP1luci + pUT651
b. pMET7-mLR F3 Del1 + pMG1-VavS + pXP2d2-rPAP1luci + pUT651
c. pMET7-mLR F3 De12 + pMG1-VavS + pXP2d2-rPAP1luci + pUT651
d. pMET7-mLR F3 Del3 + pMG1-VavS + pXP2d2-rPAP1luci + pUT651
The results are presented as fold induction compared to the non-stimulated negative control.
(L: stimulation with leptin; NC: non-stimulated negative control)

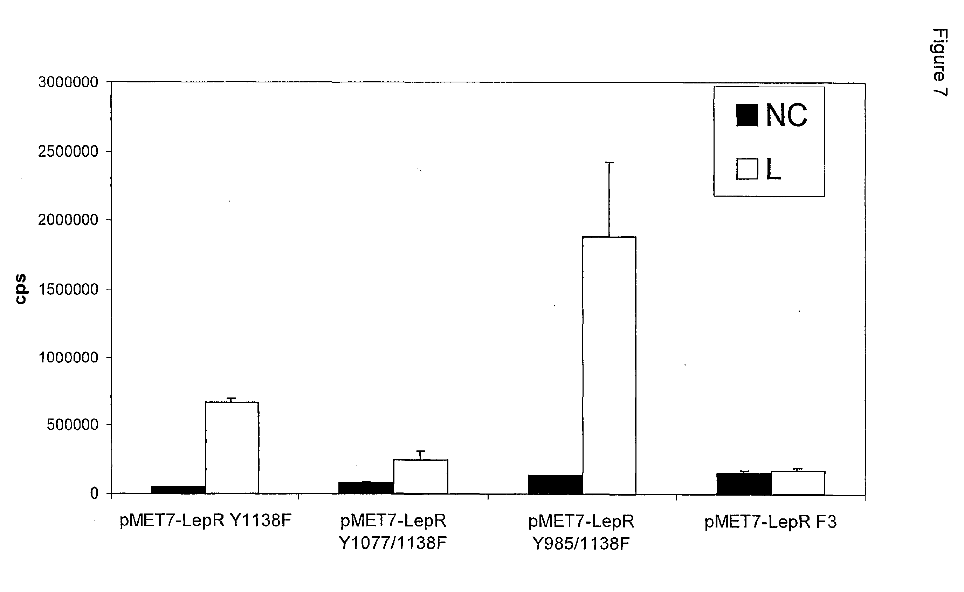

### Figure 7: CIS2 binds to both the Y985 and Y1077 motifs in the leptin receptor

HEK293T cells were transiently cotransfected with the pXP2-rPAP1luci, pMG1-CIS2 and pMET7-LR mutants as indicated. 36 hours post transfection, cells were stimulated with leptin for 24 hours (open bars) or were left untreated (solid bars). Luciferase activity is indicated as the mean of triplicate measurements +/- SD/ β-galactosidase activity from pUT561 was used to normalize for variations in transfection afficiencies. CPS: light counts per second.
(L: stimulation with leptin; NC: non-stimulated negative control)

### Examples

### Materials and methods to the examples

### Antibodies, growth factors and peptides

Monoclonal anti-FLAG antibody M2 was obtained from Sigma. Mouse recombinant leptin was purchased from R&D Systems. Peptides were synthesized using standard Fmoc-amino-acid solid phase chemistry on an Applied Biosystems Model 431A peptide synthesizer. Biotin was esterified manually following similar procedures as on the synthesizer, and added to the reaction mixture. Incorporation of phosphotyrosine was realized by using a protected Fmoc-tyrosine-phosphobenzylester (Novabiochem). After purification, mass of the peptides was confirmed by mass spectrometry. Sequences of the peptides used were biotin-QRQPSVK(p)Y₉₈₅ATLVSNDK and biotin-NHREKSVC(p)Y₁₀₇₇LGVTSVNR.

### Vectors

### Generation of the Leptin receptor mutants, the LepR F3 deletion and mLR F3 point mutants

Generation of the leptin receptor mutants was described by Eyckerman *et al.* (1999). The mutant leptin receptor (long isoform) is indicated as mLR. MLR F3 is the mutant where all the cytoplasmic residues are mutated to phenylalanine.

Through site-directed mutagenesis (Quikchange^{™}, Stratagene) three deletion and one point mutant of the mLR F3, cloned in the pMET7 expression vector, were constructed. The deletion mutants LR F3 Del1 (aa: 1-1103), LR F3 Del2 (aa: 1-1050) and LR F3 Del3 (aa: 1-952) were created by mutating Ala on position 1104 (primer combinations MBU-O-993 and MBU-O-994), Ser on position 1051 (primer combination MBU-O-885 and MBU-O-886), and Cys on position 953 (primer combination MBU-O-887 and MBU-O-888), to a STOP-codon, respectively. In each case an extra EcoRV restriction site was built in simultaneously to distinguish mutated from parental DNA.

### Construction of pMET7fSOCS3

Rat SOCS3 cDNA was amplified using
5'-GAAGATCTGTGCGCCATGGTCACCCACAGCAAGTT and
5'-GCTCTAGATTTTGCTCCTTAAAGTGGAGCATCATA as forward and reverse primer respectively, and using mRNA from leptin-stimulated PC12 cells as template. cDNA was prepared using a standard RT procedure with Superscript Reverse Transcriptase (LifeTechnologies). Amplification was realized using Pfu polymerase (Stratagene). The SOCS3 fragment was reamplified using forward primer
5'-GCGAGATCTCAGAATTCGTCACCCACAGCAAGTTTCC and the reverse primer described above, which allows Bglll-Xbal based cloning in a pMET7 variant containing a N-terminal FLAG tag sequence (MDYKDDDDK), resulting in pMET7fSOCS3.

### Construction of pMG1-CIS

CIS constructs were generated in the pMET7 vector, under control of the SRα promoter. Using site-directed mutagenesis (Quikchange^{™}, Stratagene) a unique Apal restriction site was generated between the SRα promoter and the EcoRI site in the pMET7mcs construct, a pMET7-derivative containing a multiple cloning site. The 158 amino acid C-terminal part of human gp130 was amplified using primers 5'GACGGGCCCGCCACCATGGATTACAAGGATGACGACGATAAGATCTCGACCGT GGTACACAGTGGC and 5'GCGAATTCCGAACCGCCCTGAGGCATGTAGCCGCC. The forward primer also encodes the FLAG-tag sequence (MDYKDDDDK) and contains Apal and Bglll sites. The reverse primer contains a GGS hinge sequence and an EcoRI restriction site allowing Apal-EcoRI based cloning in the pMET7 construct, resulting in the pMG1 basic vector.

A fragment of the SV40-T antigen (amino acid 261 - 708) was amplified using primers 5'GCGAATTCGAAGCAGAGGAAACTAAACAAGTG and 5'CGTCTAGAGCGGCCGCA GATCTCGAGTCGCGATTATGTTTCAGGTTCAGGGGGAG. The N-terminal part of SV40-T containing the nuclear localisation signal was deleted to prevent nuclear shuttling. The forward primer contains an EcoRI site and the reverse primer contains a stop codon and Nrul, Xhol, BgIII, Notl and Xbal restriction sites. Ligating this fragment in the pMG1 vector results in the pMG1-SVT vector.

Murine CIS was amplified with forward primer 5'GCGGAATTCGTCCTCTGCGTACAG GGATC and with reverse primer 5'GCCTCTAGATCAGAGTTGGAAGGGGTACTG). EcoRI-XbaI based cloning in the pMG1-SVT construct resulted in the pMG1-CIS plasmid.

### Construction of pMG1-CIS2

A fragment of human CIS2 (aa 34-198) containing the SH2 domain and the SOCS box sequence was amplified via RT-PCR using primers MBU-O-1046 and MBU-O-1047 as forward and reverse primer respectively. The forward primer contains an EcoRI site while the reverse contains the stop codon and a Notl restriction site. RNA from HEK293 cl16 cells was used as input in a one-step RT-PCR procedure (Qiagen Onestep RT-PCR kit). EcoRI-NotI based cloning in pMG1-SVT resulted in pMG1-CIS2.

### Construction of pMG1-VavS

A fragment of human Vav1 (VavS: aa 259-789) was amplified using Pfu polymerase from mRNA of the human TF1 cell line by standard RT-PCR techniques. Primers were MBU-O-737 and MBU-O-738 as forward and reverse respectively. MBU-O-737 contains an extra EcoRI allowing in frame fusion to gp130, and MBU-O-738 contains a stop codon and a Xhol recognition site. The amplified fragment was subcloned in de pCR®-Blunt vector and ligated in the pMG1 vector through an EcoRl-Xhol based exchange, resulting in pMG1-VavS.

### Construction of the reporter plasmids

The pUT651 construct expressing β-galactosidase was obtained from Eurogentec. Generation of the pGL3-rPAP1-luci construct was described Eyckerman *et al.* (1999). The full-length rPAP1 promoter fragment was excised using partial digestion with Kpnl and Xhol and ligated into the Kpnl-Xhol digested pXP2d2 vector (gift from Prof. S. Nordeen), resulting in the leptin-responsive pXP2d2-rPAP1-luci reporter construct. The pXP2d2 vector is a derivative of pXP2 that lacks potential cryptic Activator Protein 1 sites (Grimm and Nordeen, 1999).

All constructs were verified by restriction and sequence analysis.

| *Oligonucleotide table* | | |
|---|---|---|
| MBU-O-737 | hVavS primer | F 5'-GCGGAATTCAAGCTGGAGGAATGTTCTCA |
| MBU-O-738 | hVavSprimer | R 5'-GCCTCGAGTTACACGTAGTTGGCAGGGAACC |
| MBU-O-993 | mLR F3 Del1 mutagenesis | F 5'-TCCTGTGCACATTCCCATGACCATGGCTGTT CAGTGACATCA |
| MBU-O-994 | mLR F3 Del1 mutagenesis | R 5'-TGATGTCACTGAACAGCCATGGTCATGGGAA TGTGCACAGGA |
| MBU-O-885 | mLR F3 Del2 mutagenesis | F 5'-GATTTCACCACAACTTTGATATCCGGGGTTG GATGAGC |
| MBU-O-886 | mLR F3 Del2 mutagenesis | R 5'-GCTCATCCAACCCCGGATATCAAAGTTGTGG TGAAATC |
| MBU-O-887 | mLR F3 Del3 mutagenesis | F 5'-GAAAGCAGTTCTATTTGATATCGTGACCAGT GTAACAG |
| MBU-O-888 | mLR F3 Del3 mutagenesis | R 5'-CTGTTACACTGGTCACGATATCAAATAGAACT GCTTTC |
| MBU-O-924 | mLR Fall R225A/E226A mutagenesis | F 5'-CACCTCCGTCAACAGAGCGGCTAGCGGTGT G CTTTTGA CTGGTG |
| MBU-O-925 | mLR Fall R225A/E226A mutagenesis | R 5'-CACCAGTCAAAAGCACACCGCTAGCCGCTCT GTTGACG GAGGTG |
| MBU-O-1045 | CIS2 primer | F 5'-GCAGAATTCACCCTGCGGTGCCTGGAGCC |
| MBU-O-1046 | ClS2 primer | R 5'-GCTGCGGCCGCTTATACCTGGAATTTATAT TCTTCC |

### Cell lines and transfection procedures

Culture conditions and transient transfection procedures for PC12 and generation of the PC12LR8 cell line were as previously described (Eyckerman *et al.,* 1999, Waelput *et al.,* 2000).

HEK293T cells were maintained in a 10% CO₂ humidified atmosphere at 37°C, and were grown using DMEM with 4500 mg/l glucose, 10% foetal bovine serum and 50 µg/ml gentamycin (all from LifeTechnologies). Typically, 4.10⁵ cells were seeded the day before transfection in a 6-well plate and transfected overnight with approximately 2µg plasmid DNA using a standard calcium phosphate precipitation procedure. One day after transfection, cells were resuspended with Cell Dissociation Agent (LifeTechnologies), seeded in a black well plate (Costar), and stimulated overnight with 100 ng/ml leptin, or left unstimulated.

### Reporter assays and Northern blot

Luciferase assays, binding assays using Leptin-SEAP, and Northern blot hybridizations with SOCS3 and actin probes were described previously by Eyckerman *et al.* (1999). β-galactosidase activity was measured using the Galacto-Star™ chemiluminescent detection kit (Tropix) and a Topcount Chemiluminescence Counter (Packard).

### Western blot analysis and phosphopeptide affinity chromatography

Approximately 10⁶ HEK293T cells were lysed in 150 µl 2x loading buffer. After sonication, 30 µl was loaded on a 10% polyacrylamide gel. After overnight blotting, FLAG-tagged SOCS3 was revealed using a 1/2500 dilution of anti-FLAG antibody. Blotting efficiency was checked using PonceauS staining (Sigma). For phosphopeptide affinity chromatography, approximately 3.10⁷ HEK293T cells transiently transfected as indicated were lysed in lysis buffer (20mM Hepes pH7; 1mM MgCl₂; 10mM KCl; 0,5mM DTT; 150mM NaCl; 0,5% NP40; 1mM NaVO₄; 5mM NaF; 20% glycerol; Complete^{™} Protease Inhibitor Cocktail [Roche]). Precipitated material was cleared by 5 min. centrifugation at 10000g. To eliminate a-specific interactions, supernatants were brought on a precolumn containing Sepharose 4B beads, prior to phosphopeptide affinity chromatography on streptavidin-coupled agarose beads (Sigma). Peptide concentrations were determined by a colorimetric assay using alkaline hydrolysis and a ninhydrine reagent. 50 µl of streptavidin-agarose slurry was incubated with 5 nmoles of peptide for each reaction. Cleared lysate was incubated for 2 hours at 4°C under slow stirring. After incubation, beads were washed four times with lysis buffer and resuspended in 2x loading buffer.

### Example 1: SOCS3 binds to the conserved Y1077 region.

The observation that most human obese patients show elevated levels of leptin suggests the existence of a so-called leptin resistance (Maffei *et al.,* 1995). SOCS3, a potential mediator of this resistance (Bjorbaek *et al.,* 1998) is a member of a family of SH2 domain containing proteins mostly involved in negative regulation of signal transduction pathways (Hilton *et al.,* 1998).

Through representational difference analysis, a PCR-based differential expression screening, in PC12 cells expressing the mouse leptin receptor, several leptin-induced transcripts, including SOCS3, were identified. In order to check the inhibitory role of SOCS3 on leptin signaling in the cell systems used, an expression vector, containing rat SOCS3 was generated. Transient expression of SOCS3 in PC12 LR8 cells, a PC12 clone that stably expresses the leptin receptor, together with the pGL3-rPAP1luci construct leads to marked inhibition of leptin-mediated reporter induction (Fig. 1). The rPAP1 promoter is derived from the rat Pancreatitis Associated Protein1 gene that shows strong induction in PC12 cells upon treatment with leptin plus forskolin (Waelput *et al.,* 2000). Similar results were obtained in HEK293T cells transiently transfected with the leptin receptor, SOCS3 and the pXP2d2-rPAP1luci constructs. The pXP2d2-rPAP1luci construct showed in HEK293T cells a 4 times increased signal/background ratio and a higher reproducibility in comparison to the pGL3-based reporter construct. Taken together, these findings confirm the inhibitory activity of SOCS3 in our cell-based reporter systems.

The murine leptin receptor contains three tyrosine residues within its cytoplasmic domain (Fig.2A). Y1138 is situated within a box3 or STAT3 recruitment motif and is critical for leptin-mediated gene induction. Previously, a critical role for Y985 and to a lesser extent also for Y1077 in the murine leptin receptor in a negative feedback signal was shown (Eyckerman *et al.,* 1999). To assess the effect of Y to F mutations within the leptin receptor on SOCS3 expression itself, Northern blot analysis was performed using PC12 cells transiently transfected with leptin receptor mutants. 48 hours after transfection, cells were stimulated for 24 hours with leptin (100 ng/ml) or were left untreated (Fig. 2B). Previously obtained results indicate that SOCS3 transcription is rapidly induced in PC12 cells with an optimum around 30 minutes, but that weak SOCS3 expression persists until later time points (Waelput *et al.,* 2000). Results shown in Fig. 2B imply that during this late induction phase, mutation of Y985 results in a strong up-regulation of SOCS3 transcription. This occurs only in conjunction with Y1138, a STAT3 activation site, indicating that SOCS3 expression is STAT-dependent, as has been shown before for signaling via the IL-6 and leukemia inhibitory factor receptors (Auernhammer *et al.,* 1999, Schmitz *et al.,* 2000). Absence of the Y1077 site does not lead to altered SOCS3 mRNA expression, but lack of both sites results in a further increased induction level above what is observed for the Y985 mutant alone. These results are in line with the previous results for metallothionein II mRNA regulation (Eyckerman *et al.,* 1999). A possible explanation for the elevated SOCS3 expression level could be the loss of negative feedback via either SOCS3 itself, or alternatively via the SH2 containing phosphatase SHP-2 (Carpenter *et al.,* 1998, Li and

Friedman, 1999).

Both SOCS3 and the Cytokine Inducible SH2 containing protein (CIS), another member of the SOCS family, were shown to bind activated receptors. SOCS3 binds to the gp130 signaling component of the IL-6 complex (Nicholson *et al.,* 2000, Schmitz *et al*., 2000) , the erythropoietin receptor (Sasaki *et al.,* 2000) and the insulin receptor (Emanuelli *et al.,* 2000), while CIS binds to the erythropoietin receptor, the β common chain (Yoshimura *et al.,* 1995) but also to the leptin receptor (see example 2). Based on its functional similarity with the gp130 chain, we tested the leptin receptor for interaction with SOCS3. Leptin receptor variants containing Y to F mutations at positions Y985, Y1077, or at both sites, were tested in a functional assay based on rPAP1-induction. Signaling via the leptin receptor variants was analyzed upon transient transfection in HEK293T cells with the pXP2d2-rPAP1luci and the FLAG-tagged pMET7-fSOCS3 expression construct. Luciferase activity data were normalized by co-transfection with pUT651 and a β-galactosidase activity assay (Fig. 3B). Expression of leptin receptor variants and SOCS3 were confirmed respectively by a binding assay using a leptin-SEAP fusion protein (Fig. 3A) and Western blot analysis using an anti-FLAG antibody (Fig. 3C). The results indicated a strong inhibitory activity of SOCS3 (80-90% inhibition) when either the wild type or the mutant Y1077F leptin receptor was expressed, and a moderate inhibition (30-50% inhibition) upon expression of a Y985F receptor variant. In case of the double Y985/1077F mutant no inhibition was observed.

The interaction sites of SOCS3 with the leptin receptor were confirmed using a biochemical approach. FLAG-tagged SOCS3 was expressed upon transient transfection of pMET7-fSOCS3 in HEK293T cells, and was analyzed for binding to (phospho)-tyrosine containing peptides matching the two motifs within the leptin receptor. Lysates of approximately 3x10⁷ transfected cells were incubated with biotinylated peptides encompassing residues Y985 or Y1077 in the leptin receptor. Western blot analysis using an anti-FLAG antibody showed clear and specific binding of SOCS3 to the phosphorylated Y985 peptide, while non-phosphorylated peptide did not bind any SOCS3 protein. SOCS3 also binds specifically to the phosphorylated Y1077 peptide but apparently with a much lower affinity confirming its accessory role in SOCS3 mediated inhibition (Fig.4).

Taken together, these findings suggest that both tyrosines are involved in SOCS3 recruitment to the activated leptin receptor complex although binding to the Y1077 position apparently occurs at significantly lower efficiency suggesting an accessory role for this site. This observation could explain why, at late time points post stimulation, the single Y1077 mutant does not lead to detectable differences on induction of metallothionein II and SOCS3 genes when compared to the wild type receptor, whereas a more pronounced induction is observed when the double Y985/1077F mutant is compared to the Y985F receptor variant (Fig. 2). In line with these findings, recruitment of SOCS3 at the single phosphorylated Y1077 motif shows a moderate inhibition of leptin signaling (Fig.3). Perhaps this accessory role of the Y1077 motif is only functional when expression levels of SOCS3 are highly elevated, suggesting different threshold levels for leptin receptor signaling may exist. Although several groups were not able to show leptin dependent phosphorylation of the Y1077 site in the leptin receptor using anti-phosphotyrosine antibodies (Li and Friedman, 1999, Banks *et a*/*.,* 2000), our results indicate a functional phosphorylation-dependent role for this site in leptin signaling.

It is of note that a stretch of 10 amino acids downstream of the Y1077 motif is very conserved throughout evolution. This very pronounced conservation of the Y1077 motif also underscores its functional importance in leptin signaling.

### Example 2: CIS binds to the highly conserved Y985 and Y1077 motifs in the leptin receptor

CIS expression is rapidly induced by leptin, but recruitment to the leptin receptor was never shown. Using a functional assay, binding of CIS to both phosphorylated Y985 and Y1077 motifs have been demonstrated. Leptin receptor mutants were used that contained the Y1138F mutation, resulting in lack of STAT (Baumann *et al.,* 1996) and rPAP1 promoter (Eyckerman *et al.,* 1999) activation. Y1138F receptor mutants containing additional Y to F substitutions at positions Y985, Y1077, or at both, were transiently cotransfected with the gp130-CIS construct and the pXP2d2-rPAP1-luci and pUT651 reporter vectors. Normalised luciferase data are shown in Figure 5 and indicate comparable functional recruitment of CIS to both single Y985 and Y1077 tyrosine motifs within the leptin receptor. In line with the use of both sites, a stronger signal is obtained for the single Y1138F mutant receptor. No luciferase activity is induced in case of cells transfected with pMET7-LR-F3 or with empty vector. No difference in expression levels of the mutant receptors was observed.

Mutual comparison and cross-species relationship of the amino acid contexts of the Y985 and Y1077 residues support the functional data on CIS recruitment on both sites.

### Example 3: Vav is recruited by the conserved Y1077 region, even without phosphorylation of the tyrosine residue.

The functionality of the mLR F3 deletions were tested by following transfections in Hek293T cells:
a. pMET7-mLR F3 + pMG1-VavS + pXP2d2-rPAP1luci + pUT651
b. pMET7-mLR F3 Del1 + pMG1-VavS + pXP2d2-rPAP1luci + pUT651
c. pMET7-mLR F3 Del2 + pMG1-VavS + pXP2d2-rPAP1luci + pUT651
d. pMET7-mLR F3 Del3 + pMG1-VavS + pXP2d2-rPAP1luci + pUT651

Transfection was performed as follows. A 300 µl precipitation mixture was prepared containing 3.05 µg DNA (0.05 µg of pUT651; 1 µg of the each other vector). 200 µl of this mixture was added to 4.10⁵ cells for 18 hours. After one wash with PBS-A, cells were further incubated in 3 ml DMEM medium for 24 hours. Cells were then resuspended with 200 µl cell dissociation agent, and DMEM medium was added to a total volume of 2 ml. 50 µl of this cell suspension was transferred into a 96 well plate for each transfection and stimulation with leptin (final concentration 100 ng/ml) was performed in triplicate. 24 hours after stimulation, luciferase and β-galactosidase measurements were performed as described above.

As shown in figure 6, the results illustrate that, from the three deletion mutants, only the mLR F3 Del1 (transfection b) shows a significant induction of the rPAP1 promoter. Transfections c and d, compared to the mLR F3 (transfection a) lack induction of luciferase activity with leptin.

### Example 4: CIS2 binds to both the Y985 and Y1077 motifs in the leptin receptor

A similar functional assay in HEK293T cells as in example 2 is performed. Variant leptin receptor mutants were co-transfected with the pMG1-CIS2 construct and both the pXP2d2rPAP1luci and pUT651 reporters. Figure 7 shows normalized luciferase data. No difference in expression levels of the mutant receptors was observed. Data shown is the mean of triplicate measurements. CIS2 binds predominantly to the Y1077 site while binding to the Y985 site is less pronounced. No significant luciferase induction is observed when the LR-F3 mutant was transfected.

### References

- Attoub, S., Noe, V., Pirola, L., Bruyneel, E., Chastre, E., Mareel, M., Wymann, M.P. and Gespach, C. (2000) FASEB J. 14, 2329-2338.
- Auernhammer, C.J., Bousquet, C. and Melmed, S. (1999) Proc.Natl.Acad.Sci.U.S.A 96, 6964-6969.
- Banks, A.S., Davis, S.M., Bates, S.H. and Myers, M.G., Jr. (2000) J.Biol.Chem. 275, 14563-14572.
- Baumann, H., Morella, K.K., White, D.W., Dembski, M., Bailon, P.S., Kim, H., Lai, C.F. and Tartaglia, L.A. (1996) Proc.Natl.Acad.Sci.U.S.A. 93, 8374-8378.
- Bjorbaek, C., Elmquist, J.K., Frantz, J.D., Shoelson, S.E. and Flier, J.S. (1998) Mol.Cell 1, 619-625.
- Bjorbaek, C., Uotani, S., da Silva, B. and Flier, J.S. (1997) J.Biol.Chem. 272, 32686-32695.
- Carpenter, L.R., Farruggella, T.J., Symes, A., Karow, M.L., Yancopoulos, G.D. and Stahl, N. (1998) Proc.Natl.Acad.Sci.U.S.A. 95, 6061-6066.
- Chen, H., Charlat, O., Tartaglia, L.A., Woolf, E.A., Weng, X., Ellis, S.J., Lakey, N.D., Culpepper, J., Moore, K.J., Breitbart, R.E., Duyk, G.M., Tepper, R.I. and Morgenstern, J.P. (1996) Cell 84, 491-495.
- Cohen, B., Novick, D. and Rubinstein, M. (1996) Science 274, 1185-1188.
- El Haschimi, K., Pierroz, D.D., Hileman, S.M., Bjorbaek, C. and Flier, J.S. (2000) J.Clin.Invest 105, 1827-1832.
- Emanuelli, B., Peraldi, P., Filloux, C., Sawka-Verhelle, D., Hilton, D. and Van Obberghen, E. (2000) J.Biol.Chem. 275, 15985-15991.
- Emilsson, V., Arch, J.R., de Groot, R.P., Lister, C.A. and Cawthorne, M.A. (1999) FEBS Lett. 455, 170-174.
- Eyckerman, S., Waelput, W., Verhee, A., Broekaert, D., Vandekerckhove,J . and Tavernier, J. (1999) Eur.Cytokine Netw. 10, 549-556.
- Ghilardi, N., Ziegler, S., Wiestner, A., Stoffel, R., Heim, M.H. and Skoda, R.C. (1996) Proc.Natl.Acad.Sci.U.S.A. 93, 6231-6235.
- Grimm, S.L. and Nordeen, S.K. (1999) Biotechniques 27, 220-222.
- Heymsfield, S.B., Greenberg, A.S., Fujioka, K., Dixon, R.M., Kushner, R., Hunt,T., Lubina, J.A., Patane, J., Self, B., Hunt, P. and McCamish, M. (1999) JAMA 282, 1568-1575.
- Hilton, D.J., Richardson, R.T., Alexander, W.S., Viney, E.M., Willson, T.A., Sprigg, N.S., Starr, R., Nicholson, S.E., Metcalf, D. and Nicola, N.A. (1998) Proc.Natl.Acad.Sci. U.S.A. 95, 114-119.
- Li, C. and Friedman, J.M. (1999) Proc.Natl.Acad.Sci.U.S.A 96, 9677-9682.
- Maffei, M., Halaas, J., Ravussin, E., Pratley, R.E., Lee, G.H., Zhang, Y., Fei, H., Kim, S., Lallone, R., Ranganathan,S. and et,a. (1995) Nat.Med. 1, 1155-1161.
- Nicholson, S.E., De Souza, D., Fabri, L.J., Corbin, J., Willson, T.A., Zhang, J.G., Silva, A., Asimakis, M., Farley, A., Nash, A.D., Metcalf, D., Hilton, D.J., Nicola, N.A. and Baca,M. (2000) Proc.Natl.Acad.Sci.U.S.A 97, 6493-6498.
- Sasaki, A., Yasukawa, H., Shouda, T., Kitamura, T., Dikic, I. and Yoshimura, A. (2000) J.Biol.Chem.
- Schwartz, M.W., Peskind, E., Raskind, M., Boyko, E.J. and Porte, D. (1996) Nat.Med. 2, 589-593.
- Sierra-Honigmann, M.R., Nath, A.K., Murakami, C., Garcia-Cardena, G. Papapetropoulos, A., Sessa W.C., Madge, L.A., Schechner, J.S., Schwabb, M.B., Polverini, P.J. and Flores-Riveros, J.R. (1988) Science, 281, 1683-1686.
- Schmitz, J., Weissenbach, M., Haan, S., Heinrich, P.C. and Schaper, F. (2000) J.Biol.Chem. 275, 12848-12856.
- Takahashi, Y., Okimura, Y., Mizuno, I., lida, K., Takahashi, T., Kaji, H., Abe, H. and Chihara, K. (1997) J.Biol.Chem. 272, 12897-12900.
- Tartaglia, L.A., Dembski, M., Weng, X., Deng, N., Culpepper, J., Devos, R., Richards, G.J., Campfield, L.A., Clark, F.T. and Deeds, J. (1995) Cell 83, 1263-1271.
- Vaisse, C., Halaas, J.L., Horvath, C.M., Darnell, J.E., Jr., Stoffel, M. and Friedman, J.M. (1996) Nat.Genet. 14, 95-97.
- Waelput, W., Verhee, A., Broekaert, D., Eyckerman, S., Vandekerckhove, J., Beattie, J.H. and Tavernier, J. (2000) Biochem. J., 348, 55-61.
- Yasukawa, H., Misawa, H., Sakamoto, H., Masuhara, M., Sasaki, A., Wakioka, T., Ohtsuka, S., Imaizumi, T., Matsuda, T., Ihle, J.N. and Yoshimura, A. (1999) EMBO J. 18, 1309-1320.
- Yoshimura, A., Ohkubo, T., Kiguchi ,T., Jenkins, N.A., Gilbert, D.J., Copeland, N.G., Hara, T. and Miyajima, A. (1995) EMBO J. 14, 2816-2826.
- Zakrzewska, K.E., Cusin, I., Sainsbury, A., Rohner, J.F. and Jeanrenaud, B. (1997) Diabetes 46, 717-719.
- Zhang, J.G., Farley, A., Nicholson, S.E., Willson, T.A., Zugaro, L.M., Simpson, R.J., Moritz, R.L., Cary, D., Richardson, R., Hausmann, G., Kile, B.J., Kent, S.B., Alexander, W.S., Metcalf, D., Hilton, D.J., Nicola, N.A. and Baca, M. (1999) Proc.Natl.Acad.Sci.U.S.A. 96, 2071-2076.
- Zhang, Y., Proenca, R., Maffei, M., Barone, M., Leopold, L. and Friedman, J.M. (1994) Nature 372, 425-432.

## Claims

1. A functional fragment of a receptor, consisting of maximal 50 amino acids, comprising a sequence selected from the group consisting of SEQ ID N° 1, SEQ ID N° 2, SEQ ID N°3 and SEQ ID N° 4.

2. A functional fragment of a receptor, consisting of SEQ ID N° 5

3. A functional fragment of a receptor, consisting of SEQ ID N° 6

4. The functional fragment according to claim 1 or 2, involved in Vav signaling.

5. The functional fragment according to claim 1, whereby said sequence is SEQ ID N° 1 or SEQ ID N° 2, involved in Vav. SOCS3 or CIS signaling.

6. The functional fragment according to claim 3, involved in Vav, SOCS3 or CIS signaling.

7. A functional fragment according to any of the claims 1-6 for use as a medicament.

8. Use of a functional fragment according to claim 7, for the preparation of a medicament to treat food intake disorders, pathological angiogenesis, and cancer.

9. Use of a functional fragment according to any of the claims 1-6 to screen compounds that interfere with the binding of said functional fragment with a signaling molecule.

10. Use of a functional fragment according to claim 9, whereby said signaling molecule is Vav, SOCS3 or CIS.

## Patentansprüche

1. Funktionelles Fragment eines Rezeptors, das aus höchstens 50 Aminosäuren besteht und eine Sequenz umfasst, die aus der Gruppe bestehend aus SEQ ID Nr. 1, SEQ ID Nr. 2, SEQ ID Nr. 3 und SEQ ID Nr. 4 ausgewählt ist.

2. Funktionelles Fragment eines Rezeptors, das aus SEQ ID Nr. 5 besteht.

3. Funktionelles Fragment eines Rezeptors, das aus SEQ ID Nr. 6 besteht.

4. Funktionelles Fragment nach Anspruch 1 oder 2, das an der Vav-Signalvermittlung beteiligt ist.

5. Funktionelles Fragment nach Anspruch 1, wobei die Sequenz SEQ ID Nr. 1 oder SEQ ID Nr. 2 ist und das an der Vav-, SOCS3- oder CIS-Signalvermittlung beteiligt ist.

6. Funktionelles Fragment nach Anspruch 3, das an der Vav-, SOCS3- oder CIS-Signalvermittlung beteiligt ist.

7. Funktionelles Fragment nach einem der Ansprüche 1 - 6 zur Verwendung als ein Arzneimittel.

8. Verwendung eines funktionellen Fragments nach Anspruch 7 zur Herstellung eines Arzneimittels zur Behandlung von Nahrungsaufnahmestörungen, pathologischer Angiogenese und Krebs.

9. Verwendung eines funktionellen Fragments nach einem der Ansprüche 1 - 6 zum Screenen auf Verbindungen, die die Bindung des funktionellen Fragments mit einem Signalstoff stören.

10. Verwendung eines funktionellen Fragments nach Anspruch 9, wobei der Signalstoff Vav, SOCS3 oder CIS ist.

## Revendications

1. Fragment fonctionnel d'un récepteur, constitué de 50 acides aminés maximum, comprenant une séquence sélectionnée parmi le groupe constitué de SEQ ID N° 1, SEQ ID N° 2, SEQ ID N° 3 et SEQ ID N° 4.

2. Fragment fonctionnel d'un récepteur, constitué de SEQ ID N° 5.

3. Fragment fonctionnel d'un récepteur, constitué de SEQ ID N° 6.

4. Fragment fonctionnel selon la revendication 1 ou 2, impliqué dans la signalisation de Vav.

5. Fragment fonctionnel selon la revendication 1, moyennant quoi ladite séquence est SEQ ID N° 1 ou SEQ ID N° 2, impliqué dans la signalisation de Vav, SOCS3 ou CIS.

6. Fragment fonctionnel selon la revendication 3, impliqué dans la signalisation de Vav, SOCS3 ou CIS.

7. Fragment fonctionnel selon l'une quelconque des revendications 1 à 6 destiné à être utilisé comme médicament.

8. Utilisation d'un fragment fonctionnel selon la revendication 7 pour la préparation d'un médicament pour traiter les troubles de la prise alimentaire, l'angiogenèse pathologique et le cancer.

9. Utilisation d'un fragment fonctionnel selon l'une quelconque des revendications 1 à 6 pour cribler des composés qui interfèrent avec la liaison dudit fragment fonctionnel avec une molécule de signalisation.

10. Utilisation d'un fragment fonctionnel selon la revendication 9, moyennant quoi ladite molécule de signalisation est Vav, SOCS3 ou CIS.


### SEQUENCE LISTING

<110> VLAAMS INTERUNIVERSITAIR INSTITUUT VOOR BIOTECHNOLOGIE VZW
<120> FUNCTIONAL FRAGMENT OF A RECEPTOR
<130> JTA/TYR/V076
<150> EP 00204001.2
   <151> 2000-11-14
<150> US 60/248,970
   <151> 2000-11-15
<160> 30
<170> Patent In version 3.1
<210> 1
   <211> 6
   <212> PRT
   <213> Artificial Sequence
<220>
   <223> Description of Artificial Sequence: functional fragment of the le ptin receptor
<400> 1
   Tyr Leu Gly Val Thr Ser
   1               5
<210> 2
   <212> PRT
   <213> Artificial Sequence
<220>
   <223> Description of Artificial Sequence: functional fragment of the le ptin receptor
<400> 2
<210> 3
   <212> PRT
   <213> Artificial Sequence
<220>
   <223> Description of Artificial Sequence: functional fragment of the le ptin receptor
<400> 3
<210> 4
   <212> PRT
   <213> Artificial Sequence
<220>
   <223> Description of Artificial Sequence: functional fragment of the le ptin receptor
<400> 4
<210> 5
   <211> 20
   <212> PRT
   <213> Artificial Sequence
<220>
   <223> Description of Artificial Sequence: functional fragment of the le ptin receptor
<220>
   <221> MISC_FEATURE
   <222> (2) .. (4)
   <223> X from position 2 to 4 may be any amino acid
<220>
   <221> MISC_FEATURE
   <222> (7) .. (7)
   <223> X from position 7 may be a Val, Ile or Leu
<220>
   <221> MISC_FEATURE
   <222> (8)..(8)
   <223> X from position 8 may be a Cys or Tyr
<220>
   <221> MISC_FEATURE
   <222> (12) .. (12)
   <223> X from position 12 may be a Val or Ile
<220>
   <221> MISC_FEATURE
   <222> (15) .. (15)
   <223> X from position 15 may be a Val or Ile
<220>
   <221> MISC_FEATURE
   <222> (16) .. (17)
   <223> X from position 16 to 17 may be any amino acid
<400> 5
   Asn Xaa Xaa Xaa Lys Ser Xaa Xaa Phe Leu Gly Xaa Thr Ser Xaa Xaa
   1               5                   10                  15
   Xaa Arg Glu Ser
               20
<210> 6
   <211> 20
   <212> PRT
   <213> Artificial Sequence
<220>
   <223> Description of Artificial Sequence: functional fragment of the le ptin receptor
<220>
   <221> MISC_FEATURE
   <222> (2)..(4)
   <223> X from position 2 to 4 may be any amino acid
<220>
   <221> MISC_FEATURE
   <222> (7)..(7)
   <223> X from position 7 may be a Val or Ile
<220>
   <221> MISC_FEATURE
   <222> (8) .. (8)
   <223> X from position 8 may be a Cys or Tyr
<220>
   <221> MISC_FEATURE
   <222> (12)..(12)
   <223> X from position 12 may be a Val or Ile
<220>
   <221> MISC_FEATURE
   <222> (15) ..(15)
   <223> X from position 15 may be a Val or Ile
<220>
   <221> MISC_FEATURE
   <222> (16).. (17)
   <223> X from position 16 to 17 may be any amino acid
<400> 6
   Asn Xaa Xaa Xaa Lys Ser Xaa Xaa Tyr Leu Gly Xaa Thr Ser Xaa Xaa
   1               5                   10                  15
   Xaa Arg Glu Ser
               20
<210> 7
   <211> 16
   <212> PRT
   <213> Artificial Sequence
<220>
   <223> Sequence starts with biotin-Q
<220>
   <221> MISC_FEATURE
   <223> Tyr985 is phosphorylated
<400> 7
   Gln Arg Gln Pro Ser Val Lys Tyr Ala Thr Leu Val Ser Asn Asp Lys
   1               5                   10                  15
<210> 8
   <211> 17
   <212> PRT
   <213> Artificial Sequence
<220>
   <223> Sequence starts with biotin-N
<220>
   <221> MISC_FEATURE
   <223> Tyr1077 is phosphorylated
<400> 8
   Asn His Arg Glu Lys Ser Val Cys Tyr Leu Gly Val Thr Ser Val Asn
   1               5                   10                  15
   Arg
<210> 9
   <211> 35
   <212> DNA
   <213> Artificial Sequence
<220>
   <223> Description of Artificial Sequence: forward primer for amplificat ion of Rat SOCS3 cDNA
<400> 9
   gaagatctgt gcgccatggt cacccacagc aagtt 35
<210> 10
   <211> 35
   <212> DNA
   <213> Artificial Sequence
<220>
   <223> Description of Artificial Sequence : reverse primer for amplificat ion of Rat SOCS3 cDNA
<400> 10
   gctctagatt ttgctcctta aagtggagca tcata 35
<210> 11
   <211> 37
   <212> DNA
   <213> Artificial Sequence
<220>
   <223> Description of Artificial Sequence: forward primer for reamplific ation of the SOCS3 fragment
<400> 11
   gcgagatctc agaattcgtc acccacagca agtttcc 37
<210> 12
   <211> 9
   <212> PRT
   <213> Artificial Sequence
<220>
   <223> Description of Artificial Sequence: N-terminal FLAG-tag sequence
<400> 12
   Met Asp Tyr Lys Asp Asp Asp Asp Lys
   1               5
<210> 13
   <211> 66
   <212> DNA
   <213> Artificial Sequence
<220>
   <223> Description of Artificial Sequence: primer used for amplification of the 158 AA C-terminal part of human gp130
<400> 13
   gacgggcccg ccaccatgga ttacaaggat gacgacgata agatctcgac cgtggtacac     60
   agtggc                                                                66
<210> 14
   <211> 35
   <212> DNA
   <213> Artificial Sequence
<220>
   <223> Description of Artificial Sequence: primer used for amplification of the 158 AA C-terminal part of human gp130
<400> 14
   gcgaattccg aaccgccctg aggcatgtag ccgcc 35
<210> 15
   <211> 32
   <212> DNA
   <213> Artificial Sequence
<220>
   <223> Description of Artificial Sequence: primer for amplification of a fragment of the SV40-T antigen
<400> 15
   gcgaattcga agcagaggaa actaaacaag tg 32
<210> 16
   <211> 55
   <212> DNA
   <213> Artificial Sequence
<220>
   <223> Description of Artificial Sequence: primer for amplification of a fragment of the SV40-T antigen
<400> 16
   cgtctagagc ggccgcagat ctcgagtcgc gattatgttt caggttcagg gggag 55
<210> 17
   <211> 29
   <212> DNA
   <213> Artificial Sequence
<220>
   <223> Description of Artificial Sequence: forward primer for murine CIS amplification
<400> 17
   gcggaattcg tcctctgcgt acagggatc 29
<210> 18
   <211> 30
   <212> DNA
   <213> Artificial Sequence
<220>
   <223> Description of Artificial Sequence: reverse primer for murine CIS amplicication
<400> 18
   gcctctagat cagagttgga aggggtactg 30
<210> 19
   <211> 29
   <212> DNA
   <213> Artificial Sequence
<220>
   <223> Description of Artificial Sequence: primer MBU-O-737
<400> 19
   gcggaattca agctggagga atgttctca 29
<210> 20
   <211> 31
   <212> DNA
   <213> Artificial Sequence
<220>
   <223> Description of Artificial Sequence: primer MBU-O-738
<400> 20
   gcctcgagtt acacgtagtt ggcagggaac c 31
<210> 21
   <211> 42
   <212> DNA
   <213> Artificial Sequence
<220>
   <223> Description of Artificial Sequence: primer MBU-O-993
<400> 21
   tcctgtgcac attcccatga ccatggctgt tcagtgacat ca 42
<210> 22
   <211> 42
   <212> DNA
   <213> Artificial Sequence
<220>
   <223> Description of Artificial Sequence: primer MBU-O-994
<400> 22
   tgatgtcact gaacagccat ggtcatggga atgtgcacag ga 42
<210> 23
   <211> 38
   <212> DNA
   <213> Artificial Sequence
<220>
   <223> Description of Artificial sequence: primer MBU-O-885
<400> 23
   gatttcacca caactttgat atccggggtt ggatgagc 38
<210> 24
   <211> 38
   <212> DNA
   <213> Artificial Sequence
<220>
   <223> Description of Artificial Sequence: primer MBU-O-886
<400> 24
   gctcatccaa ccccggatat caaagttgtg gtgaaatc 38
<210> 25
   <211> 38
   <212> DNA
   <213> Artificial Sequence
<220>
   <223> Description of Artificial Sequence: primer MBU-O-887
<400> 25
   gaaagcagtt ctatttgata tcgtgaccag tgtaacag 38
<210> 26
   <211> 38
   <212> DNA
   <213> Artificial Sequence
<220>
   <223> Description of Artificial Sequence: primer MBU-O-888
<400> 26
   ctgttacact ggtcacgata tcaaatagaa ctgctttc 38
<210> 27
   <211> 44
   <212> DNA
   <213> Artificial Sequence
<220>
   <223> Description of Artificial Sequence: primer MBU-O-924
<400> 27
   cacctccgtc aacagagcgg ctagcggtgt gcttttgact ggtg 44
<210> 28
   <211> 44
   <212> DNA
   <213> Artificial Sequence
<220>
   <223> Description of Artificial Sequence: primer MBU-O-925
<400> 28
   caccagtcaa aagcacaccg ctagccgctc tgttgacgga ggtg 44
<210> 29
   <211> 29
   <212> DNA
   <213> Artificial Sequence
<220>
   <223> Description of Artificial Sequence: primer MBU-O-1045
<400> 29
   gcagaattca ccctgcggtg cctggagcc 29
<210> 30
   <211> 36
   <212> DNA
   <213> Artificial Sequence
<220>
   <223> Description of Artificial Sequence: primer MBU-O-1046
<400> 30
   gctgcggccg cttatacctg gaatttatat tcttcc 36